(19) Europäisches Patentamt
European Patent Office
Office européen des brevets

(11) Publication number: 0 284 587 A2

(12) # EUROPEAN PATENT APPLICATION

(21) Application number: 88850105.3

(22) Date of filing: 28.03.88

(51) Int. Cl.$^4$: C 07 K 7/10
G 01 N 33/569

(30) Priority: 27.03.87 SE 8701294 18.05.87 US 51726

(43) Date of publication of application:
28.09.88 Bulletin 88/39

(84) Designated Contracting States:
AT BE CH DE FR GB IT LI NL SE

(71) Applicant: VIROVAHL S.A.
Baarerstrasse 43
CH-6300 Zug (CH)

(72) Inventor: Vahlne, Anders
Igelkottssstigen 14A
S-430 80 Hovas (SE)

Svennerholm, Bo
Jakobsdalsgatan 8
S-412 68 Göteborg (SE)

Rymo, Lars
Hällekullavägen 17
S-430 80 Hovas (SE)

Jeansson, Stig
Föreningsgatan 33
S-411 27 Göteborg (SE)

Horal, Peter
Orangerigatan 21B
S-412 66 Göteborg (SE)

(74) Representative: Roth, Ernst Adolf Michael et al
GÖTEBORGS PATENTBYRA AB Box 5005
S-402 21 Göteborg (SE)

(54) **Synthetic peptide antigens for the detection of HIV-1 infection.**

(57) Synthetic peptides corresponding to regions of immunologically reactive proteins of HIV-1 are provided. The peptides which are immunologically reactive with HIV-1 specific antibodies are useful in assays for detection of HIV-1 infection or exposure and in compositions to elicit the production of antibodies against HIV-1 in animals including man.

EP 0 284 587 A2

**Description**

SYNTHETIC PEPTIDE ANTIGENS FOR THE DETECTION OF HIV-1 INFECTION

BACKGROUND OF THE INVENTION

The present invention relates to synthetic peptide antigens, the sequences of which correspond to regions of HIV-1 proteins, and their use as diagnostic reagents to detect the presence of antibodies to HIV-1. The peptides may also be useful as immunogens in compositions to elicit the production of antibodies against HIV-1.

HIV-1 (human immunodeficiency virus-1) is the name given to a group of highly related viruses which have been identified as the primary etiologic agent of the acquired immunodeficiency syndrome (AIDS) in humans. HIV-1 which is also known as HTLV-III, LAV and ARV, is a major worldwide health problem. Since HIV-1 was first identified as the etiologic agent of AIDS, substantial progress has been made in studies on the virus per se and mechanisms by which the virus causes disease and in the development of diagnostic tests to detect exposure to the virus or infection.

Methods for detecting HIV-1 infection, in general, measure exposure to the virus by detecting and quantifying antibodies to HIV-1 antigens in blood, sera, and blood-derived products. Such methods are used to aid diagnosis of AIDS and ARC (AIDS-Related Complex) and to screen blood and blood products for previous exposure to HIV-1.

The diagnosis of HIV-1 infections and screening of blood for exposure to HIV-1 is generally performed by enzyme-linked immunosorbent assay (ELISA) techniques in order to detect the presence of antibodies to immunogenic components of HIV-1 in a test sample. Other methods involve the use of Western blotting techniques to detect HIV-1 specific antibodies in test samples (Schorr et al., N. Engl. J. Med. (1985) 313:384-385). In general, almost any known immunoassay, such as radioimmunoassays, can be adapted, by use of specific reagents, for the detection of HIV-1 and antibodies thereto.

The source of antigens in the first generation of these assays has generally been antigenic proteins obtained from HIV-1 produced by human helper T-lymphoblastoid cell lines, such as H9 (Popovic et al., Science (1984) 224:497-500; Gallo et al. Science (1984) 224:500-503). Even though these first generation ELISA tests for HIV-1 are quite sensitive and specific, the use of antigens obtained from live virus preparations has several significant drawbacks.

The production of HIV-1 per se in continuous cell lines must be performed in high risk (P3 containment) laboratories due to the danger to investigators who may become adversely exposed to the virus. In addition, there are clearly false negative and false positive results that have been obtained with ELISA tests using whole virus antigens. Western blot analyses, using electroblotted whole virus antigen, provide greater specificity but are more laborious and time-consuming then ELISA tests (Schorr et al., supra). Furthermore, since H9 and other HIV-1 producing cells are human cell lines, viral antigens preparation obtained from these cell lines, unless exhaustively purified, may be contaminated with normal cellular antigens, such as HLA antigens, which could produce false positive reactions in an ELISA test (Kuhnl et al., Lancet I (1985): 1222-1223; Hunter et al., Lancet II (1985): 397.)

Exhaustive purification of viral antigens from cell lines can also conceivably destroy immunogenicity of immunologically important proteins or otherwise inactivate antigens, thereby producing reagents that result in false negative reactions. In addition, false negative reactions using antigens obtained from intact virus preparations may occur because of steric hinderance whereby antibodies to one viral antigen cannot react with their specific antigen because the reaction is blocked by the presence of other viral antigens and antibodies in the reaction mixture.

Second generation ELISA tests to detect HIV-1 infection have employed immunologically important viral proteins that have been produced by cloning portions of the HIV-1 genome in bacteria. The viral etiologic agent of AIDS (i.e., those viruses previusly named HTLV-III, LAV and ARV) from a variety of sources has been isolated, cloned and the nucleotide sequence determined (Popovic et al., Science (1984) 224: 497; Gallo et al., Science (1984) 224: 500; Schupbach et al., Science (1984) 224: 503; Shaw et al., Science (1984) 226: 1165; Barre-Sinoussi et al., Science (1983) 220: 868; Levy et al., Science (1984) 225: 840; Ratner et al., Nature (1985) 313: 277; Muesing et al., Nature (1985) 313: 450; Wain-Hobson et al., Cell (1985) 40:9; Sanchez-Pescador et al., Science (1985) 227: 484; Shaw et al., Adv. Intern. Med. (1984) 30: 1).

As shown in Fig. 1, HIV-1 is a relatively complex retrovirus containing at least seven genes. The viral structural genes designated gag, pol and env respectively code for the viral core proteins, reverse transcriptase, and the viral glycoproteins of the viral envelope. The other genes shown in Fig. 1 are accessory genes involved in viral replication. The gag and env genes encode polyproteins, i.e., the proteins synthesized from each of these genes are post translationally cleaved into several smaller proteins. Previous studies have shown that the proteins coded by the gag and especially the env regions of the HIV-1 genome are immunologically important, since antibodies to the products of the gag and env genes are found in the sera of AIDS and ARC patients.

The env gene encodes a glycoprotein (gp160) with an apparent molecular weight (Mr) of about 160,000 daltons which is post synthetically cleaved into two glycoproteins, gp120 and gp41, of Mr 120,000 and Mr 41,000 respectively. Glycoprotein gp120 is apparently the external protein of the viral envelope, while gp41 appears to be a transmembrane protein. Both gp120 and gp41 are immunogenic, with antibodies to both proteins readily detectable in AIDS and ARC patient sera. Because antibodies to gp120 and gp160 in sera of AIDS and ARC patients, as well as asymptomatic individuals infected with the virus, are neutralizing, i.e., inhibit binding of the virus, gp120, gp160 or portions thereof are candidates for a subunit vaccine. The transmembrane glycoprotein gp41 is the HIV-1 antigen most consistently recognized by antibodies in AIDS and ARC patient sera (Allan et al., Science (1985) 228: 1091-1094; Barin et al., Science (1985) 228: 1094-1096). In addition, antibodies in patient sera also recognize epitopes of the viral core proteins encoded by the gag gene.

Immunologically important HIV-1 antigens for use in diagnosis and as potential vaccine compositions have been prepared by cloning portions of the HIV-1 genome in various expression systems such as bacteria, yeast or vaccinia (See, e.g., Cabradilla et al., Biotechnology (1986)4: 129-133; Chang et al., Biotechnology (1985) 3: 905-909; Putney, et al., Science (1986) 234: 1392-1395; Kieny et al. Biotechnology (1986) 4: 790-795). HIV-1 antigens produced by recombinant DNA methods, however, must still be exhaustively purified to avoid false positive reactions in the ELISA due to any antibody reactivity to antigens of the expression system which may contaminate the HIV-1 antigen preparation. Also, denaturation of HIV-1 antigens during purification may destroy important antigen activity.

For example, Chang et al., supra and Cabridilla et al., supra produced portions of gp41 by recombinant DNA techniques which were then used to detect the presence of antibodies in sera of AIDS/ARC patients by ELISA. Both studies reported false negative reactions in 2/132 sera (Chang et al) and 2/127 sera (Cabradilla et al.). While the studies documented a significant improvement in the percentage of false negatives and false positives over ELISA tests based on human cell line-derived antigens, because of the nature of AIDS, a diagnosis that is as close to 100% accurate as possible is desirable and other reagents have been developed to try to achieve this.

Protein antigens contain a number of epitopes or antigenic determinants which are the regions of the proteins which comprise the binding sites for specific antibodies. In general, proteins antigens contain between 5 to 10 epitopes, each of which containing a sequence of 6 to 8 amino acids. Epitopes can be either continuous, in which the 6 to 8 amino acids are present in sequence, or discontinuous, in which the amino acids that form the epitope are brought together by the three dimensional folding of the protein. Even though an epitope constitutes only a relatively few amino acids, its reactivity with an antibody is influenced by the amino acids in the protein which surround the epitope.

Studies aimed at mapping antigenic sites or epitopes of proteins have been aided by the use of synthetic peptides corresponding to various regions of the proteins of interest (See e.g., Lerner et al., in The Biology of Immunological Disease: A Hospital Practice Book, (1983) Dixon and Fisher, eds., pp. 331-338; Lerner, Adv. Immunol. (1984) 36: 1). In addition to their usefulness in epitope mapping studies, synthetic peptides, if encompassing major antigenic determinants of a protein, have potential as vaccines and diagnostic reagents. Synthetic peptide antigens have several advantages in regard to specific antibody production and reactivity. The sequence of the synthesized peptide can be selected from the amino acid sequence as actually determined by amino acid sequencing of a protein or predicted from the DNA sequence coding for the protein. The use of specific synthetic peptides eliminate the need for using the full-length protein in the production of or assay for specific antibodies. Furthermore, the solid phase peptide synthetic techniques of Merrifield and coworkers allow for essentially unlimited quantities of the peptide of interest to be chemically produced. (See, e.g., Erickson and Merrifield in The Proteins, 3rd Edit. (1976), Vol. 2, Academic Press, New York, Chapter 3). The availability of automated peptide synthesizers has further advanced such techniques.

Several publications have presented data showing immunologic reactivity of selected synthetic peptides corresponding to antigenic proteins of HIV-1. In one study, a peptide having the amino acid sequence Tyr-Asp-Arg-Pro-Glu-Gly-Ile-Glu-Glu-Glu-Gly-Gly-Glu-Arg-Asp-Arg-Asp-Arg-Ser-Gly-Cys which corresponds to amino acid residues 735-752 of HIV-1 was synthesized. (Kennedy et al., Science (1986) 231:1556-1559). This peptide which is a portion of gp41 was used to immunize rabbits in an attempt to elicit a neutralizing antibody response to HIV-1. Furthermore, several sera from AIDS patients known to contain anti-gp41 antibodies were weakly reactive with this peptide, thus indicating that this peptide contains at least one epitope recognized, to some extent, by antibodies to native gp160/gp41.

Recent work has been directed at providing synthetic peptides for AIDS diagnostics, in addition to developing vaccine compositions. In one study (Wang et al., Proc. Natl. Acad. Sci. (1986) 83:6159-6163), a 21 amino acid peptide called SM284 with the amino acid sequence Arg-Ile-Leu-Ala-Val-Glu-Arg-Tyr-Leu-Lys-Asp-Gln-Gln-Leu-Leu-Gly-Ile-Trp-Gly-Cys-Ser was synthesized. This peptide, which corresponds to amino acids 586-606 of gp160 and comprises an antigenic segment of gp41, was reactive with HIV-1 positive sera from AIDS/ARC patients by ELISA and Western blotting. The SM284 peptide compared favorably with HIV-1-derived proteins in detecting HIV-1 antibodies in an ELISA. For example, the ELISA using SM284 detected antibodies in serum samples from 96.5% of AIDS/ARC patients and 34.6% of healthy asymptomatic high risk individuals. There were no false positives in 387 sera from control individuals.

Serological and chemical analysis of the SM284 peptide and related peptides showed that certain amino acids were apparently more important than others in the antibody-antigen interaction of the peptide. Deletion of residues Arg-1, Ile-2 and Lys-10 from the peptide significantly reduced or destroyed serologic reactivity. Deletion of the Try-Gly-Cys-Ser residues at the carboxy terminal of the peptide, on the other hand, resulted in moderate loss of serologic activity, indicated that the amino terminal portion of the peptide is apparently more important than the carboxy terminal portion for immunologic reactivity.

U.S. Patent No. 4,629,783 issued December 16, 1986 to Cosand provides several immunologically reactive synthetic peptides corresponding to regions of HIV-1 proteins for detection of AIDS and AIDS-related disease. Of particular interest is peptide V (39) having the sequence Arg-Ile-Leu-Ala-Val-Glu-Arg-Tyr-Leu-Lys-Asp-Gln-Gln-Leu-Leu-Gly-Ile-Trp-Gly-Cys-Ser-Gly-Lys-Leu-Ile-Cys-X, where X is OH or $NH_2$, which corresponds to a portion of gp41 encoded by base pairs (bp) 7516-7593 of the HIV-1 genome. Peptide V (39) reacted with 23/24 (95.8%) serum samples from patients with confirmed HIV-1 infections.

## SUMMARY OF THE INVENTION

In accordance with the present invention, several novel synthetic peptides corresponding to antigenic HIV-1 proteins are provided which are useful and superior in highly selective diagnostic methods for detecting HIV-1 infections.

Novel synthetic peptide antigens corresponding to glycoprotein gp41 encoded by the HIV-1 env gene and the protein products encoded by the HIV-1 gag gene have now been found. These peptides are useful for diagnosing AIDS in suspected individuals and in methods for screening for exposure to HIV-1 in blood and blood-derived products with a high degree of reliability and very few false results.

The peptides can be used in methods of detecting antibodies to HIV-1 in samples. The methods involve contacting the sample with the peptide antigens under conditions which allow an immunological complex to form between the peptide and any HIV-1 specific antibodies in the sample. Measuring complex formation by suitable detection means indicates the presence or absence of antibodies to HIV-1 in the sample.

The novel peptides may also be used as immunogens in vaccine compositions for immunization against HIV-1 infection or for the production in animals of HIV-1 specific antibodies against HIV-1 antigens.

## BRIEF DESCRIPTION OF THE DRAWINGS

Fig. 1 is a schematic representation of the genes of HIV-1.

Fig. 2 is a schematic representation of gp41 of HIV-1 depicting the overlapping peptides synthesized in accordance with the invention and a comparison with prior art peptides corresponding to gp41.

Fig. 3 is a histogram showing ELISA values obtained using gp41A5 as antigen.

## DESCRIPTION OF THE INVENTION

The present invention provides a number of peptides of 15 to 27 amino acids in length corresponding to regions of the protein product of the entire HIV-1 gag gene and to gp41 of HIV-1 which have been synthesized and tested for immunoreactivity to HIV-1 positive serum samples obtained from the United States, United Kingdom, Israel, Africa and Sweden. The novel peptides are useful in tests to diagnose HIV-1 infection or prior exposure to the virus and as immunogens in compositions to elicit the production in animals and man of antibodies against HIV-1. Peptides corresponding to gag proteins and in particular gp41 were selected and synthesized because these HIV-1 proteins demonstrate little strain to strain variation characteristic of other immunologically important HIV-1 antigenic proteins such as gp120. The peptides encompassed by the invention comprise oligopeptides having amino acid sequences containing therein sequences which comprise continuous (linear) epitopes reactive with HIV-1 specific antibodies.

The invention thus encompasses a group of immunologically reactive peptides and functionally equivalent variants thereof which do not significantly affect the antigenic properties of the peptide corresponding to gp41 encoded by the env gene of HIV-1 (Fig. 1). The regions of gp41 corresponding to each peptide described in detail below is shown in Fig. 2. The peptides were all synthesized by known solid phase peptide synthesis techniques (see e.g., Merrifield and Barany, The Peptides: Analysis, Synthesis, Biology (1980), Vol. 1, Gross and Meinenhofer, eds., Academic Press, New York, Chap. 1). The synthesis also allows for one or two amino acids not corresponding to the original protein sequence to be added to the $NH_2$- or COOH- terminus of the peptides. Such extra amino acids are useful for coupling the peptides to each other, to a large carrier protein or to a support. Amino acids that are useful for these purposes include tyrosine, lysine, glutamic acid, aspartic acid, cysteine and derivatives thereof. Additional protein modification techniques may be used, e.g., $NH_2$-acetylation or COOH-terminal amidation, to provide additional means for coupling the peptides to another protein or peptide molecule or to a support.

The novel peptides corresponding to gp41 are set forth below:

gp41A5X-Asp-Gln-Gln-Leu-Leu-Gly-Ile-Trp-Gly-Cys-Ser-Gly-Lys-Leu-Ile-Cys-Thr-Thr-Ala-Val-Pro-Trp-Asn-Y-Z, wherein X is either a H of the amino terminal $NH_2$ group of the peptide or an additional amino acid bonded to the amino terminal, $NH_2$ group of the peptide, the additional amino acid being selected to facilitate coupling the peptide to a carrier protein; Y is absent or Cys; and Z is OH or $NH_2$.

Peptide gp41A5, which corresponds to amino acids 596-618 encoded by about bp 7563 through 7632 of the HIV-1 genome corresponding to the env gene, (Wang et al., supra numbering), is a particularly preferred embodiment of the present invention. Peptide gp41A5 in which X is $H_2$, Y is Cys and Z is OH is especially preferred.

gp41CT4

The peptide gp41CT4 which corresponds to the region of the gp41 protein encoded by about bp 8173 through 8238 of the HIV-1 genome, has the formula: X-Ala-Leu-Lys-Tyr-Trp-Trp-Asn-Leu-Leu-Gln-Tyr-Trp-Ser-Gln-Glu-Leu-Lys-Asn-Ser-Ala-Val-Ser-Y-Z, wherein X, Y, and Z have the same definitions as above.

gp41CT3

The peptide gp41CT3M which corresponds to the region of gp41 encoded by about bp 8220 through 8280 of the HIV-1 env gene, has the formula: X-Lys-Asn-Ser-Ala-Val-Ser-Leu-Leu-Asn-Ala-Thr-Ala-Ile-Ala-Val-Ala-Glu-Gly-Thr-Asp-Y-Z, wherein X, Y, and Z have the same definitions as above.

gp41B1

The peptide gp41B1 which corresponds to the region of gp41 encoded by about bp 7614 through 7686 of the HIV-1 env gene has the formula: X-Thr-Ala-Val-Pro-Trp-Asn-Ala-Ser-Trp-Ser-Asn-Lys-Ser-Leu-Glu-Gln-Ile-Trp-Asn-Asn-Met-Thr- Trp-Met-Y-Z, wherein X,Y, and Z have the same definitions as above.

gp41B3

The peptide gp41B3 which corresponds to the region of gp41 encoded by about bp7705 through 7773 of the HIV-1 genome has the formula: X-Ile-Asn-Asn-Tyr-Thr-Ser-Leu-Ile-His-Ser-Leu-Ile-Glu-Glu-Ser-Gln-Asn-Gln-Gln-Glu-Lys-Asn-Glu-Y-Z, wherein X,Y, and Z have the same definitions as above.

The invention further encompasses several immunologically reactive peptides and functionally equivalent variants thereof corresponding to regions of the protein products encoded by the gag gene of HIV-1 (Fig. 1). These novel peptides are set forth as follows:

GAG 2

The peptide GAG 2, which corresponds to the region of the gag gene product encoded by about bp 779 through 840 of the gag gene of HIV-1, has the formula: X-Pro-Arg-Thr-Leu-Asn-Ala-Trp-Val-Lys-Val-Val-Glu-Glu-Lys-Ala-Phe-Ser-Pro-Glu-Val-Y-Z, wherein X,Y and Z have the same definitions as above.

GAG 3

The peptide GAG 3, which corresponds to the region of the gag gene protein product encoded by about bp 810 through 870 of the gag gene, has the formula: X-Val-Glu-Glu-Lys-Ala-Phe-Ser-Pro-Glu-Val-Ile-Pro-Met-Phe-Ser-Ala-Leu-Ser-Glu-Gly-Y-Z, wherein X,Y and Z have the same definitions as above.

GAG 14

The peptide GAG 14, which corresponds to the region of the gag gene product encoded by about bp 1368 through 1430 of HIV-1 gag gene, has the formula: X-Glu-Met-Met-Thr-Ala-Cys-Gln-Gly-Val-Gly-Gly-Pro-Gly-His-Lys-Ala-Arg-Val-Leu-Ala-Glu-Y-Z, wherein X, Y and Z have the same definitions as above.

P17-D

The peptide P17-D, which corresponds to the region of the gag gene protein product encoded by about bp 495 through 560 of HIV-1 gag gene, has the formula: X-Ser-Glu-Gly-Cys-Arg-Gln-Ile-Leu-Gly-Gln-Leu-Gln-Pro-Ser-Leu-Gln-Thr-Gly-Ser-Glu-Glu-Leu-Y-Z, wherein X, Y and Z have the same definitions as above.

P17-F

The peptide P17-F, which corresponds to the region of the gag gene protein product encoded by about bp 588 through 647 of the HIV-1 gag gene, has the formula: X-Leu-Tyr-Cys-Val-His-Gln-Arg-Ile-Glu-Ile-Lys-Asp-Thr-Lys-Glu-Ala-Leu-Asp-Lys-Ile-Y-Z, wherein X,Y and Z have the same definitions as above.

The peptides can be used in methods for detection of antibodies to HIV-1 or HIV-1 associated antigens. Preferably the methods which use the peptides to detect the presence of HIV-1 specific antibodies in the sample involve contacting the sample with at least one of the peptides under conditions which allow the formation of an immunological complex between the peptide antigen and any antibodies to HIV-1 that may be present in the sample. The formation of an immunological complex if any, indicating the presence of antibodies to HIV-1 in the sample, is then detected and measured by suitable means.

Such methods include, inter alia, homogeneous and heterogeneous binding immunoassays, such as radioimmunoassays (RIA), ELISA and Western blot analyses. Further, the assay protocols using the novel peptides allow for competitive and non-competitive binding studies to be performed.

The peptides may be labeled (signal-generating) or unlabeled depending on the type of assay used. Labels which may be coupled to the peptides are those known in the art and include inter alia enzymes, radionuclides, fluorogenic and chromogenic substrates, cofactors, biotin/avidin, colloidal gold, and magnetic particles. Modification of the novel peptides, allows for coupling by known means to carrier proteins or peptides or to known supports, for example, polystyrene or polyvinyl microtiter plates, glass tubes or glass beads and chromatographic supports, such as paper, cellulose and cellulose derivates, and silica.

Preferred assay techniques, especially for large-scale clinical screening of patient sera and blood and blood-derived products are ELISA and Western blot techniques, ELISA tests being particularly preferred. The ELISA tests employing the peptides described above are based on those currently in use with human cell-derived or recombinant DNA-derived HIV-1 proteins or portions thereof as antigens. For use as reagents in these assays, the peptides are conveniently bonded to the inside surface of microtiter wells. The peptides may be directly bonded to the microtiter well. It has been found, however, that maximum binding of the peptides to the wells can be accomplished by pretreating the wells with polylysine prior to the addition of the peptides. Additionally, the novel peptides may be covalently attached by known means to a carrier protein, such as BSA, with the resulting conjugate being used to coat the wells. Generally the peptides were used in a concentration of between 10 to 100 µg/ml for coating, although some peptides required as much as 500 µg/ml for the assay to be successful.

Samples are then added to the peptide coated wells where an immunological complex forms if antibodies to HIV-1 are present in the sample. A signal generating means may be added to aid detection of complex formation. A detectable signal is produced if HIV-1 specific antibodies are present in the sample.

The invention is further illustrated by the following specific examples which are not intended in any way to limit the scope of the invention.

Example 1

An Applied Biosystems peptide-synthesizer Model 430 A, was utilized for the synthesis of all the peptides. Each synthesis used a p-methylbenzylhydrylamine solid phase support resin (Peptides International, Louisville, KY). The peptides were synthesized according to the Users Manual for Peptide Synthesizer Model 430A, Applied Biosystems, 1986.

All amino acids for use in synthesis contained t-butylcarbonyl groups (t-Boc) protecting the $\alpha$-$NH_2$ group and were obtained from Novabiochem AG, Switzerland. Amino acids with reactive side chain groups contained additional protective groups to prevent unwanted and undesirable side chain reactions. The individual protected amino acids used in synthesizing all of the peptides are set forth in Table 1.

Table 1

Amino Acids Used in the Synthesis of Peptides

Boc-Ala-OH
Boc-Arg (Tos)-OH
Boc-Asn-OH
Boc-Asp (OBzl)-OH
Boc-Cys (pMeOBzl)-Oh
Boc-Glu (OBzl)-OH
Boc-Gln-OH
Boc-Gly-OH
Boc-His(Tos)-OH
Boc-Ile-OH·1/2 $H_2O$
Boc-Leu-OH·$H_2O$
Boc-Lys (2-Cl-Z)-OH (cryst.)
Boc-Met-OH
Boc-Phe-OH
Boc-Pro-OH
Boc-Ser(Bzl)-OH·DCHA
Boc-Thr (Bzl)-OH
Boc-Trp (Formyl)-OH
Boc-Tyr(2-Br-Z)-OH
Boc-Val-OH

Tos = Tosyl or p-Toluene sulfonic acid
oBzl = Benzyloxy
pMeoBzl = p-Methylbenzyloxy
2-Cl-Z = Carbobenzoxy chloride
2-Br-Z = Carbobenzoxybromide

After completion of a particular synthesis, the protecting groups were removed from the synthesized peptide and the peptide cleaved from the solid support resin by treatment at 0° C with anhydrous hydrofluoric acid (HF) combining 10% anisole and 10% dimethylsulfide as scavenging agents. 2% thiocresol was also added for cysteine-containing peptides. After cleavage, the HF in the sample was purged under a stream of $N_2$, with removal of any residual HF accomplished by subjecting the sample to a vacuum at 0° C. The peptides were extracted from the resin by treatment with trifluoracetic acid (TFA) which was then removed by evaporation at room temperature. Following TFA removal, the peptides were precipitated and washed with anhydrous ether.

Prior to use in specific assays, the peptides can be further purified, if desired, by reverse phase high performance liquid chromatography (HPLC). A particularly suited column for such purification is the reverse-phase Vydek®C-18 column using a water (TFA) - acetonitrile (TFA) gradient to elute the peptides.

Example 2

Peptide gp41A5 having the amino acid sequence Asp-Gln-Gln-Leu-Leu-Gly-Ile-Trp-Gly-Cys-Ser-Gly-Lys-Leu-Ile-Cys-Thr-Thr-Ala-Val-Pro-Trp-Asn-Cys-OH was synthesized as described in Example 1 and used in an ELISA to measure its immunologic reactivity.

Polylysine at a concentration of 1 mg/ml was added to the microtiter plates and allowed to incubate for 30 minutes. The polylysine was then discarded and peptide gp41A5 was added to the wells of the plate in a concentration of from 10 to 100 µg/ml for coating. After the peptide incubated in the well for a length of time sufficient to allow the peptide to be bonded to the well, the peptide solution was removed and a solution of glutaraldyhyde, which stabilizes the peptide attachment to the wells, was added for 15 minutes. The glutaraldehyde solution was then removed, the wells washed with buffer, and a mixture of glycine and bovine serum albumin (BSA) was added which served to block unbound sites in the wells and minimize non-specific binding of antibodies during the ELISA test per se. After a final washing step, the plates were ready to use. The prepared peptide-coated microtiter plates could be stored for several months without any decrease in antigenic activity of peptide gp41A5 coated on the wells.

A convenient variation of known ELISA methods was used with the microtiter plates prepared as above. Serum samples from individuals which had been diluted 1:50 in PBS (phosphate buffered saline) containing 0.05% polyoxyethylenesorbitan monolaurate (Tween 20) and 1% BSA were added to each well and allowed to incubate for 90 minutes at 37°C in a humidified atmosphere. The diluted serum samples were then removed from the plates and the wells washed three times with PBS containing 0.05% Tween 20. A conjugated anti-human Ig antibody was then added to the wells and allowed to incubate for 90 minutes. The conjugated antibody was produced in a goat or rabbit and was specific for human IgG, IgM, immunoglobulin light chains, or combinations thereof. Preferably, alkaline-phosphatase conjugated anti human IgG (from Dakopatts) diluted 1:500 for use in PBS containing 0.05% Tween 20 and 1% BSA was used in the ELISA. After the conjugate had incubated a sufficient length of time to react with bound human antibodies, the plates were washed three times as above. In order to detect antibodies to HIV-1 in the human serum that react with the peptide gp41A5 used as the antigen, (i.e. positive reactions), a chromogenic substrate alkaline phosphatase substrate (Sigman Cat. No. 104 tablets) dissolved in a Na carbonate/MgCl buffer and adjusted to a concentration of 1 µg/ml which was cleaved by the enzyme attached to the antihuman Ig to yield a colored product was added. After incubation for approximately 40 minutes at room temperature, positive reactions indicated the presence of antibodies in the sample reactive with the antigen. A yellow to orange to reddish-brown color in each well indicating a positive reaction, was read in a spectrophotometer at 405nm to quantify the reaction. Spectrophotometric readings were adjusted to correct for background reactions.

Fig. 3 is a histogram showing antibody values obtained following four separate ELISA determinations using gp41A5 as antigen. 101 true positive (by Western blot analysis) sera from AIDS and ARC patients and 179 true negative sera were reacted in the ELISA with gp41A5. The mean cutoff for positive reactions was determined to be $O.D._{405} = 0.376$. Fig. 3 shows clearly that gp41A5 gave a positive reaction with 100% (101/101) of true positive sera and 0% (0/179) of true negative sera.

Example 3

For purposes of comparison with the novel peptides the known peptide V (39) of Cosand was synthesized as described in Example 1 in accordance with its amino acid sequence disclosed in U.S. Patent No. 4,629,783. The relative location of this peptide in gp41 is shown in Fig. 2 which also provides the relative sequence location of gp41A5 and other subject peptides corresponding to gp41 of HIV-1. Cosand reported that peptide V (39) reacted with 23/24 (95.8%) confirmed positive sera (i.e., one false negative reaction). The peptide was used in the ELISA described in Example 2 with the substitution of peptide V(39) for gp41A5.

Peptide (V)(39) produced 221/233 (94.8%) positive reactions with known HIV-1 positive serum samples. Twelve false negative reactions were seen as shown in Table 2. In addition peptide V(39) gave a false positive reaction in one of 102 (0.98%) confirmed negative sera. Serum samples were confirmed as positive and negative by Western blot analysis with HIV-1 proteins. All false positive and negative reactions were corroborated by Western blot analysis.

It is further seen in Fig. 2, that gp41A5 has a partial overlap in amino acid sequence with peptide SM284 of Wang et al., Proc. Natl. Acad. Sci. (1986) 83:6159-6163, and peptide V (39) of Cosand. Peptide gp41A5 thus contains the amino acid sequence of the carboxy terminal portion of the two known peptides plus an additional sequence corresponding to a region of gp41 to the carboxy terminal side of peptides SM284 and V(39). As discussed previously, Wang et al. considered the carboxy terminal portion of peptide SM284 to be less important immunologically when compared to the amino terminal portion of peptide SM284. Contrary to Wang et al., extending the permutation of gp41A5 to the carboxy terminus of gp41, however, resulted in a peptide having excellent antigenic properties for detection of AIDS-specific antibodies in human sera. This peptide shows both a higher degree of reactivity and greater specificity in ELISA and Western blots than both peptides V(39) and SM284.

Table 2 also clearly shows that gp41A5 reacts with all 233 (100%) confirmed positive HIV-1 sera. These results were obtained from the same 233 sera tested against peptide V(39) which yielded 12 false negative reactions. The mean cut off values for a positive reaction in the ELISA tests was an $O.D._{405}$ of about 0.3. Any reading below 0.3 was considered a negative reaction. It is readily apparent that the serum samples numbered 7, 19, 40, 44, 48, 60, 101, 482, 511, 324, 375 and 393 clearly show no reactivity with peptide V(39) but superior reactivity with gp41A5.

Table 3 provides a further comparison of the superior immunologic reactivity of gp41A5 compared with peptide V(39) of Cosand. Thirty-eight of the 233 serum samples presented in Table 2 were confirmed as positive by Western blot analysis. These positive sera plus 8 confirmed negative serum samples (by Western blot) were reacted with peptides gp41A5 and V(39) in parallel ELISA tests with positive reactions being anything greater than an $O.D._{405}$ of about 0.3. In addition, a non-HIV-1 antigen (negative antigen) was used a a control. It is readily apparent that gp41A5 produced no false negative or false positive reactions. Thus, gp41A5 surprisingly and unexpectedly gives ELISA results that are clearly 100% accurate. The diagnostic and screening capabilities of assays using gp41A5 as antigen are clearly superior to currently available assay using peptides V(39) and SM284 or any other HIV-1 antigen.

TABLE 2

Immunologic Reactivity Determined By ELISA Between HIV-1 Antibodies In 233 Confirmed Positive Serum Samples And Synthetic Peptide Antigens Corresponding To Regions Of gp41

| Serum Sample No. | Antigen | |
|---|---|---|
| | V (39) | gp41A5 |
| 4 | 0.436 | 2.809 |
| 7 | 0.137 | 1.412 |
| 12 | 0.336 | 2.222 |
| 13 | 0.836 | 2.890 |
| 17 | 0.885 | 1.527 |
| 18 | 1.336 | 2.711 |
| 19 | 0.289 | 2.922 |
| 23 | 1.706 | 2.822 |
| 24 | 0.485 | 2.010 |
| 25 | 0.484 | 2.635 |
| 26 | 1.443 | 2.600 |
| 27 | 0.810 | 2.029 |
| 29 | 1.399 | 2.459 |
| 30 | 1.889 | 2.500 |
| 31 | 0.737 | 2.343 |
| 32 | 1.244 | 2.902 |
| 33 | 1.332 | 2.871 |
| 35 | 0.938 | 2.714 |
| 37 | 1.087 | 2.859 |
| 38 | 1.028 | 2.378 |
| 39 | 1.362 | 2.529 |
| 40 | 0.227 | 1.495 |
| 41 | 1.282 | 2.692 |
| 42 | 0.731 | 2.289 |
| 43 | 0.522 | 1.978 |
| 44 | 1.199 | 2.443 |
| 45 | 1.096 | 1.844 |
| 46 | 1.456 | 2.796 |
| 47 | 0.665 | 2.636 |
| 48 | 0.195 | 2.592 |
| 49 | 1.532 | 2.809 |
| 51 | 0.712 | 2.579 |
| 53 | 1.217 | 2.714 |
| 54 | 0.602 | 2.423 |
| 55 | 1.444 | 2.567 |
| 56 | 0.832 | 2.511 |
| 57 | 1.164 | 2.468 |
| 58 | 1.516 | 2.232 |
| 59 | 1.919 | 2.726 |
| 60 | 0.219 | 1.399 |
| 78 | 0.489 | 1.826 |
| 79 | 0.927 | 2.247 |
| 101 | 0.205 | 0.863 |
| 482 | 0.120 | 1.316 |
| 511 | 0.248 | 1.036 |
| 130 | 0.346 | 1.741 |
| 134 | 1.256 | 2.425 |
| 137 | 1.095 | 2.413 |
| 140 | 0.952 | 2.364 |

TABLE 2 (Continued)

Immunologic Reactivity Determined By ELISA Between HIV-1 Antibodies In 233 Confirmed Positive Serum Samples And Synthetic Peptide Antigens Corresponding To Regions Of gp41

| Serum Sample No. | Antigen | |
|---|---|---|
| | V (39) | gp41A5 |
| 225 | 1.464 | 2.548 |
| 232 | 1.877 | 2.654 |
| 243 | 0.644 | 2.397 |
| 272 | 1.968 | 2.767 |
| 273 | 1.404 | 2.660 |
| 274 | 2.037 | 2.821 |
| 275 | 1.786 | 2.609 |
| 276 | 1.900 | 2.745 |
| 277 | 1.106 | 2.243 |
| 278 | 1.203 | 2.354 |
| 279 | 0.555 | 2.579 |
| 283 | 1.293 | 2.570 |
| 285 | 0.437 | 1.977 |
| 286 | 1.017 | 2.108 |
| 287 | 0.798 | 1.845 |
| 288 | 0.875 | 2.569 |
| 289 | 1.544 | 2.572 |
| 291 | 0.728 | 2.561 |
| 292 | 0.690 | 2.032 |
| 293 | 1.096 | 2.466 |
| 294 | 0.861 | 2.228 |
| 295 | 1.631 | 2.696 |
| 297 | 1.598 | 2.764 |
| 298 | 1.204 | 2.636 |
| 300 | 1.854 | 2.636 |
| 301 | 0.796 | 2.431 |
| 302 | 1.065 | 2.623 |
| 303 | 0.733 | 2.381 |
| 305 | 1.375 | 2,686 |
| 306 | 1.745 | 2.696 |
| 307 | 1.239 | 2.383 |
| 308 | 1.142 | 2.687 |
| 312 | 1.291 | 2.553 |
| 313 | 1.100 | 2.152 |
| 314 | 0.456 | 1.266 |
| 317 | 0.669 | 1.051 |
| 318 | 1.329 | 1.616 |
| 319 | 1.255 | 2.008 |
| 320 | 1.024 | 1.941 |
| 321 | 1.493 | 2.347 |
| 322 | 1.092 | 1.850 |
| 323 | 1.292 | 2.077 |
| 324 | 0.281 | 1.299 |
| 330 | 0.725 | 1.483 |
| 332 | 0.683 | 1.385 |
| 333 | 1.620 | 1.901 |
| 334 | 0.923 | 1.579 |
| 336 | 1.309 | 1.951 |
| 337 | 1.258 | 2.166 |
| 340 | 1.194 | 1.689 |

TABLE 2 (Continued)

Immunologic Reactivity Determined By ELISA Between HIV-1 Antibodies In 233 Confirmed Positive Serum Samples And Synthetic Peptide Antigens Corresponding To Regions Of gp41

| Serum Sample No. | Antigen | |
|---|---|---|
| | V (39) | gp41A5 |
| 341 | 1.290 | 1.763 |
| 357 | 1.138 | 1.754 |
| 358 | 0.738 | 1.733 |
| 359 | 0.599 | 1.033 |
| 360 | 0.528 | 0.840 |
| 363 | 0.711 | 1.291 |
| 365 | 1.390 | 1.963 |
| 366 | 0.821 | 1.682 |
| 368 | 0.665 | 1.417 |
| 371 | 1.183 | 1.585 |
| 372 | 1.075 | 1.593 |
| 373 | 0.799 | 1.711 |
| 374 | 1.046 | 1.650 |
| 375 | 0.280 | 0.957 |
| 377 | 1.065 | 1.480 |
| 378 | 1.261 | 1.644 |
| 379 | 0.610 | 1.242 |
| 380 | 1.205 | 1.763 |
| 381 | 1.321 | 1.645 |
| 383 | 0.552 | 1.146 |
| 384 | 0.620 | 1.029 |
| 385 | 0.973 | 1.618 |
| 386 | 1.687 | 1.646 |
| 387 | 0.695 | 1.189 |
| 388 | 0.928 | 1.396 |
| 389 | 0.938 | 1.544 |
| 390 | 0.679 | 1.190 |
| 391 | 0.709 | 1.250 |
| 392 | 0.629 | 1.014 |
| 393 | 0.195 | 0.874 |
| 394 | 0.622 | 1.244 |
| 395 | 0.799 | 1.404 |
| 396 | 0.736 | 1.416 |
| 398 | 0.766 | 1.647 |
| 400 | 1.210 | 1.621 |
| 401 | 0.482 | 1.073 |
| 402 | 0.614 | 1.302 |
| 403 | 0.498 | 1.145 |
| 406 | 1.570 | 1.600 |
| 407 | 0.742 | 1.186 |
| 408 | 0.398 | 0.801 |
| 411 | 1.154 | 1.603 |
| 412 | 1.209 | 0.864 |
| 413 | 0.557 | 1.220 |
| 414 | 1.170 | 1.787 |
| 416 | 0.526 | 1.326 |
| 417 | 0.473 | 1.201 |
| 419 | 1.020 | 1.592 |
| 420 | 0.637 | 0.944 |
| 422 | 0.742 | 1.479 |

TABLE 2 (Continued)

Immunologic Reactivity Determined By ELISA Between HIV-1 Antibodies In 233 Confirmed Positive Serum Samples And Synthetic Peptide Antigens Corresponding To Regions Of gp41

| Serum Sample No. | Antigen | |
|---|---|---|
| | V (39) | gp41A5 |
| 426 | 0.921 | 1.381 |
| 427 | 0.548 | 1.116 |
| 428 | 0.355 | 1.184 |
| 429 | 0.381 | 1.051 |
| 431 | 0.542 | 1.198 |
| 432 | 1.192 | 1,633 |
| 433 | 1.402 | 1.793 |
| 434 | 0.637 | 1.196 |
| 435 | 1.017 | 1.951 |
| 436 | 1.155 | 1.512 |
| 437 | 0.465 | 1.408 |
| 438 | 0.517 | 0.572 |
| 439 | 0.483 | 0.422 |
| 440 | 1.345 | 1.380 |
| 441 | 0.618 | 1.238 |
| 442 | 0.660 | 1.088 |
| 443 | 0.638 | 1.288 |
| 444 | 0.604 | 0.737 |
| 445 | 1.187 | 1.462 |
| 446 | 1.731 | 0.662 |
| 447 | 2.737 | 2.913 |
| 448 | 2.150 | 2.936 |
| 449 | 1.580 | 2.901 |
| 450 | 2.287 | 2.956 |
| 451 | 1.645 | 2.777 |
| 452 | 1.741 | 2.636 |
| 453 | 1.998 | 2.922 |
| 454 | 1.756 | 2.860 |
| 455 | 2.590 | 3.010 |
| 456 | 1.022 | 2.936 |
| 457 | 1.055 | 2.724 |
| 458 | 1.706 | 2.868 |
| 459 | 1.133 | 2.849 |
| 460 | 1.611 | 2.636 |
| 461 | 1.809 | 2.881 |
| 462 | 1.417 | 2.788 |
| 463 | 1.034 | 1.536 |
| 464 | 2.027 | 2.936 |
| 477 | 0.586 | 1.843 |
| 478 | 0.651 | 1.783 |
| 480 | 0.678 | 1.554 |
| 510 | 0.807 | 2.180 |
| 512 | 1.583 | 2.670 |
| 513 | 1.592 | 2.796 |
| 514 | 1.922 | 2.812 |
| 515 | 2.102 | 2.769 |
| 516 | 1.434 | 1.918 |
| 517 | 0.369 | 0.791 |
| 518 | 1.143 | 2.498 |
| 520 | 0.593 | 1.923 |

TABLE 2 (Continued)

Immunologic Reactivity Determined By ELISA
Between HIV-1 Antibodies In 233 Confirmed
Positive Serum Samples And Synthetic Peptide
Antigens Corresponding To Regions Of gp41

| Serum Sample No. | Antigen | |
| --- | --- | --- |
| | V (39) | gp41A5 |
| 521 | 2.418 | 2.831 |
| 522 | 1.107 | 2.493 |
| 523 | 0.509 | 1.043 |
| 524 | 0.825 | 2.621 |
| 525 | 0.829 | 1.993 |
| 526 | 1.456 | 2.612 |
| 527 | 2.311 | 2.849 |
| 528 | 0.679 | 2.026 |
| 529 | 0.530 | 2.631 |
| 530 | 1.655 | 2.745 |
| 531 | 1.177 | 2.739 |
| 532 | 0.384 | 1.617 |
| 533 | 1.085 | 2.698 |
| 534 | 1.852 | 2.834 |
| 535 | 1.412 | 2.744 |
| 536 | 0.350 | 1.093 |
| 537 | 0.905 | 2.764 |
| 538 | 1.147 | 2.590 |
| 539 | 0.219 | 0.407 |
| 540 | 0.643 | 2.644 |

TABLE 3

ELISA Tests Comparing Specificity And Selectivity of Synthetic Peptide Antigens

| SERUM SAMPLE NO. | WB | Antigen gp41A5 | V (39) | Negative antigen** |
|---|---|---|---|---|
| 4 | + | 2.809* | 0.436* | 0.084* |
| 7 | + | 1.412 | 0.137 | 0.066 |
| 12 | + | 2.222 | 0.336 | 0.087 |
| 19 | + | 2.922 | 0.289 | 0.071 |
| 25 | + | 2.635 | 0.484 | 0.071 |
| 31 | + | 2.343 | 0.737 | 0.101 |
| 40 | + | 1.495 | 0.227 | 0.090 |
| 42 | + | 2.289 | 0.731 | 0.140 |
| 43 | + | 1.978 | 0.522 | 0.062 |
| 44 | + | 2.443 | 1.199 | 0.085 |
| 48 | + | 2.592 | 0.195 | 0.073 |
| 60 | + | 1.399 | 0.219 | 0.084 |
| 78 | + | 1.826 | 0.489 | 0.219 |
| 101 | + | 0.863 | 0.205 | 0.072 |
| 482 | + | 1.316 | 0.120 | 0.100 |
| 511 | + | 1.036 | 0.248 | 0.067 |
| 140 | + | 2.369 | 0.952 | U.081 |
| 277 | + | 2.243 | 1.106 | 0.260 |
| 285 | + | 1.977 | 0.437 | 0.057 |
| 298 | + | 2.636 | 1.204 | 0.046 |
| 324 | + | 1.299 | 0.281 | 0.057 |
| 375 | + | 0.957 | 0.280 | 0.066 |
| 393 | + | 0.874 | 0.195 | 0.067 |
| 416 | + | 1.326 | 0.526 | 0.082 |
| 428 | + | 1.184 | 0.355 | 0.142 |
| 446 | + | 0.612 | 1.731 | 0.215 |
| 457 | + | 2.724 | 1.055 | 0.205 |
| 477 | + | 1.843 | 0.586 | 0.104 |
| 478 | + | 1.783 | 0.651 | 0.069 |
| 517 | + | 0.791 | 0.369 | 0.029 |
| 520 | + | 1.923 | 0.593 | 0.088 |
| 523 | + | 1.093 | 0.509 | 0.080 |
| 524 | + | 2.621 | 0.825 | 0.056 |
| 525 | + | 1.993 | 0.809 | 0.067 |
| 532 | + | 1.617 | 0.384 | 0.082 |
| 536 | + | 1.093 | 0.350 | 0.086 |
| 539 | + | 0.407 | 0.219 | 0.051 |
| 540 | + | 2.644 | 0.643 | 0.056 |
| 39508 | - | 0.154 | 0.153 | 0.087 |
| 39509 | - | 0.191 | 0.166 | 0.043 |
| 39510 | - | 0.156 | 0.085 | 0.044 |
| 39511 | - | 0.241 | 0.177 | 0.028 |
| 39512 | - | 0.101 | 0.144 | 0.093 |
| 39513 | - | 0.178 | 0.123 | 0.048 |
| 39514 | - | 0.101 | 0.118 | 0.051 |
| 39515 | - | 0.086 | 0.252 | 0.063 |

* Spectrophotometric readings, $O.D._{405}$
** Non HIV-1 Antigen

Cut off = Mean $O.D._{405}$ of Negative Sera + 3xSD

Cut off A5 = 0.151 + 3x0.053 = 0.310 ($O.D._{405}$)
Cut off V(39) = 0.152 + 3x0.050 = 0.302 ($O.D._{405}$)
WB = Western Blot using HIV-1 antigens

Example 4

A peptide A5-Trunc(a) missing the carboxyl terminal portion of gp41A5 (see Fig. 2) was also synthesized as described in Example 1 and used in ELISA tests as described in Example 2 but substituting peptide A5-Trunc(a) for gp41A5 to measure antigenic reactivity. It is readily seen from the ELISA results presented in Table 4 that the carboxy terminal portion of gp41A5 is clearly required for the high degree of reactivity and specificity of this peptide.

TABLE 4

Reactivity Of Antibodies in Serum Samples
With Synthetic Peptide Antigens Corresponding
to HIV-1 Antigens

| SERUM SAMPLE NO. | WB | Antigen gp41A5 | V (39) | A5-Trunc(a) | Negative antigen |
|---|---|---|---|---|---|
| 4 | + | 2.809* | 0.436* | 0.905* | 0.084* |
| 7 | + | 1.412 | 0.137 | 0.164 | 0.066 |
| 12 | + | 2.222 | 0.336 | 0.583 | 0.087 |
| 19 | + | 2.922 | 0.289 | 0.850 | 0.071 |
| 25 | + | 2.635 | 0.484 | 0.532 | 0.071 |
| 31 | + | 2.343 | 0.737 | 0.444 | 0.101 |
| 40 | + | 1.495 | 0.227 | 0.547 | 0.090 |
| 42 | + | 2.289 | 0.731 | 0.420 | 0.140 |
| 43 | + | 1.978 | 0.522 | 0.144 | 0.062 |
| 44 | + | 2.443 | 1.199 | 0.406 | 0.085 |
| 48 | + | 2.592 | 0.195 | 0.601 | 0.073 |
| 60 | + | 1.399 | 0.219 | 0.098 | 0.084 |
| 78 | + | 1.826 | 0.489 | 0.485 | 0.219 |
| 101 | + | 0.863 | 0.205 | 0.187 | 0.072 |
| 482 | + | 1.316 | 0.120 | 0.605 | 0.100 |
| 511 | + | 1.036 | 0.248 | 0.139 | 0.067 |
| 140 | + | 2.369 | 0.952 | 0.318 | 0.081 |
| 277 | + | 2.243 | 1.106 | 0.307 | 0.260 |
| 285 | + | 1.977 | 0.437 | 0.553 | 0.057 |
| 298 | + | 2.636 | 1.204 | 0.229 | 0.046 |
| 324 | + | 1.299 | 0.281 | 0.363 | 0.057 |
| 375 | + | 0.957 | 0.280 | 0.631 | 0.066 |
| 393 | + | 0.874 | 0.195 | 0.254 | 0.067 |
| 416 | + | 1.326 | 0.526 | 0.166 | 0.082 |
| 428 | + | 1.184 | 0.355 | 0.303 | 0.142 |
| 446 | + | 0.612 | 1.731 | 0.310 | 0.215 |
| 457 | + | 2.724 | 1.055 | 0.326 | 0.205 |
| 477 | + | 1.843 | 0.586 | 0.672 | 0.104 |
| 478 | + | 1.783 | 0.651 | 0.386 | 0.069 |
| 517 | + | 0.791 | 0.369 | 0.125 | 0.029 |
| 520 | + | 1.923 | 0.593 | 0.990 | 0.088 |
| 523 | + | 1.093 | 0.509 | 0.272 | 0.080 |
| 524 | + | 2.621 | 0.825 | 0.557 | 0.056 |
| 525 | + | 1.993 | 0.809 | 0.485 | 0.067 |
| 532 | + | 1.617 | 0.384 | 0.432 | 0.082 |
| 536 | + | 1.093 | 0.350 | 0.532 | 0.086 |
| 539 | + | 0.407 | 0.219 | 0.147 | 0.051 |
| 540 | + | 2.644 | 0.643 | 0.334 | 0.056 |
| 39508 | - | 0.154 | 0.153 | 0.209 | 0.087 |
| 39509 | - | 0.191 | 0.166 | 0.179 | 0.043 |
| 39510 | - | 0.156 | 0.085 | 0.160 | 0.044 |
| 39511 | - | 0.241 | 0.177 | 0.151 | 0.028 |
| 39512 | - | 0.101 | 0.144 | 0.156 | 0.093 |
| 39513 | - | 0.178 | 0.123 | 0.131 | 0.048 |
| 39514 | - | 0.101 | 0.118 | 0.130 | 0.051 |
| 39515 | - | 0.086 | 0.252 | 0.216 | 0.063 |

Cut off = Mean $O.D._{405}$ of Negative Sera + 3xSD
Cut off A5 = 0.151 + 3x0.053 = 0.310

Cut off V(39) = 0.152 + 3x0.050 = 0.302
Cut off A5-Trunc(a) = 0.166 + 3x0.033 = 0.265
WB = Western Blot

* Spectrophotometric reading, $O.D._{405}$
** Non-HIV-1 Antigen

Example 5

Peptide gp41CT4 having the amino acid sequence set forth above was synthesized as in Example 1 and was tested in the ELISA described in Example 2 (substituting gp41CT4 for gp41A5) against 105 HIV-1 positive sera (all of which reacted with gp41A5). The peptide, at a concentration of between 10 and 100 µg/ml was coated onto microtiter plates as described in Example 2. As shown in Table 5, this peptide reacted with 68/105 (64.8%) positive sera. No false positive reactions were seen when this peptide was tested against 28 confirmed negative sera.

Example 6

Peptide gp41CT3 having the amino acid sequence set forth above was synthesized as in Example 1 and coated onto microtiter plates as described in Example 2 (substituting gp41CT3 for gp41A5). As shown in Table 5, gp41CT3 reacted with 38/80 (48.8%) of the confirmed positive serum samples in the ELISA assay described in Examples 2 and 3. The peptide produced only one false positive reaction when tested against 20 confirmed negative sera.

Example 7

Peptide gp41B1 synthesized as in Example 1 at a concentration of from 10 to 100 µg/ml was coated into microtiter plates as described above and used in the ELISA described in Example 2 (substituting gp41B1 for gp41A5). This peptide reacted with 45/95 (47.4%) of the confirmed HIV-1 positive serum samples (Table 5). No false positive reactions were seen with two negative sera.

Example 8

Peptide gp41B3 synthesized as described in Example 1 was coated at a concentration of from 10 to 1200 µg/ml onto microtiter plates. It yielded positive reactions with 22/32 (68.8%) of the confirmed positive HIV-1 serum samples in the ELISA described in Examples 2 and 3 (Table 5). No false positive reactions were seen with two confirmed negative sera.

TABLE 5

Reactivity Of Synthetic Peptides Corresponding To Regions Of HIV-1 gp41 With Antibodies Against HIV-1 In Confirmed Positive Sera

| | | Peptide (Fig. 2) | | | | | |
|---|---|---|---|---|---|---|---|
| Serum | A5 | A4 | B1 | B3 | B4 | CT3 | CT4 |
| 1 | + | − | + | + | + | + | (+) |
| 2 | + | | + | + | − | + | (+) |
| 3 | + | + | + | + | + | + | + |
| 4 | + | + | + | + | + | + | − |
| 5 | + | + | + | + | − | + | (+) |
| 6 | + | + | + | + | + | − | + |
| 7 | + | | + | − | − | − | − |
| 8 | + | + | + | + | (+) | − | + |
| 9 | + | + | + | + | (+) | + | + |
| 10 | + | + | + | (+) | + | − | − |
| 11 | + | | + | | − | − | + |
| 12 | + | | + | | − | + | + |
| 13 | + | + | + | + | + | + | + |
| 14 | + | | + | + | + | (+) | + |
| 15 | + | − | + | − | − | − | + |
| 16 | + | + | + | + | + | + | + |
| 17 | + | | | | | + | + |
| 18 | + | | | | | (+) | − |
| 19 | + | | | | | + | + |
| 20 | + | − | + | + | + | + | + |
| 21 | + | + | + | + | − | + | |
| 22 | + | − | + | − | + | | |
| 23 | + | | | | | − | + |
| 24 | + | | | + | | − | − |
| 25 | + | | | − | | − | + |
| 26 | + | | | − | | − | + |
| 27 | + | | | − | | − | + |
| 28 | + | − | (+) | + | − | | |
| 29 | + | | | | | − | + |
| 30 | + | | | | | + | + |
| 31 | + | | | | | + | + |
| 32 | + | | | | | + | − |
| 33 | + | | | | | + | + |
| 34 | + | + | (+) | + | − | + | + |
| 35 | + | | | − | | + | + |
| 36 | + | + | + | + | + | | |
| 37 | + | | | + | | + | + |
| 38 | + | | | − | | + | + |
| 39 | + | | | (+) | | − | − |
| 40 | + | | | (+) | | − | − |
| 41 | + | | | | | + | + |
| 42 | + | | | | | + | + |
| 43 | + | | | | | − | + |
| 44 | + | | | | | + | + |
| 45 | + | | | (+) | | + | + |
| 46 | + | | | + | | + | + |
| 47 | + | | | + | | + | + |
| 48 | + | | | | | − | − |
| 49 | + | | | | | + | + |
| 50 | + | + | − | + | + | + | + |

TABLE 5 (Continued)

Reactivity Of Synthetic Peptides Corresponding To Regions Of HIV-1 gp41 With Antibodies Against HIV-1 In Confirmed Positive Sera

| Serum | A5 | Peptide (Fig. 2) A4 | B1 | B3 | B4 | CT3 | CT4 |
|---|---|---|---|---|---|---|---|
| 51 | + | | | | | | |
| 52 | + | + | (+) | (+) | + | | + |
| 53 | + | | | | | + | + |
| 54 | + | | | | | | + |
| 55 | + | | | | | + | + |
| 56 | + | | | | | + | − |
| 57 | + | | | | | | + |
| 58 | + | | | | | + | − |
| 59 | + | | | (+) | | − | (+) |
| 60 | + | | | | | + | − |
| 61 | + | | | | | − | − |
| 62 | + | | | | | + | − |
| 63 | + | − | (+) | + | | | − |
| 64 | + | (+) | + | | | | − |
| 65 | + | (+) | + | + | | | − |
| 66 | + | + | + | | | | + |
| 67 | + | (+) | + | | | | − |
| 68 | + | + | + | | | | − |
| 69 | + | − | + | | | | + |
| 70 | + | (+) | + | | | | − |
| 71 | + | (+) | + | | | | + |
| 72 | + | | | | | | − |
| 73 | + | + | − | | | | − |
| 74 | + | − | − | | | | − |
| 75 | + | + | + | | | | + |
| 76 | + | − | − | | | | + |
| 77 | + | + | + | | | | − |
| 78 | + | (+) | + | | | | − |
| 79 | + | (+) | − | | | | − |
| 80 | + | − | + | | | | − |
| 81 | + | | | | | | − |
| 82 | + | | | | | | − |
| 83 | + | | | | | | + |
| 84 | + | | | | | | − |
| 85 | + | | | | | | − |
| 86 | + | | | | | | − |
| 87 | + | | | | | + | + |
| 88 | + | | | | | + | + |
| 89 | + | | | | | − | + |
| 90 | + | | | | | − | − |
| 91 | + | | | | | − | + |
| 92 | + | | | | | − | + |
| 93 | + | | | | | − | + |
| 94 | + | | | | | − | + |
| 95 | + | | | | | − | + |
| 96 | + | | | | | − | + |
| 97 | + | | | | | − | + |
| 98 | + | | | | | − | + |
| 99 | + | | | | | − | + |
| 100 | + | | | | | − | − |
| 101 | + | + | (+) | | | − | − |

TABLE 5 (Continued)

Reactivity Of Synthetic Peptides Corresponding To Regions Of HIV-1 gp41 With Antibodies Against HIV-1 In Confirmed Positive Sera

| Serum | A5 | Peptide (Fig. 2) A4 | B1 | B3 | B4 | CT3 | CT4 |
|---|---|---|---|---|---|---|---|
| 102 | + | | | | | - | - |
| 103 | + | | | | | - | + |
| 104 | + | | | | | + | + |
| 105 | + | | | | | - | + |
| 106 | + | | | | | - | + |
| 107 | + | | | | | + | + |
| 108 | + | | | | | - | + |
| 109 | + | | | | | - | + |
| 110 | + | | | | | - | - |

- = negative reaction
(+)= low positive reaction
+ = strong positive reaction

Example 9

Peptides GAG2 and GAG3 and GAG 14 were synthesized as described in Example 1. Each peptide was coated at a concentration of from 10 to 100 µg/ml onto microtiter plates and used in the ELISA described in Example 2, but substituting GAG 2, GAG 3, or GAG 14 for gp41A5 as the antigen. Peptide GAG 2 was also assayed against HIV-2 positive serum samples. The results of the assays are presented in Table 6.

TABLE 6

Immunologic Reactivity Of Peptide Antigens Corresponding To HIV-1 Gag Gene Product

| | Serum Source | | | | |
|---|---|---|---|---|---|
| Antigen | Confirmed Pos. (HIV-1) No. Reactive/No. Tested | % | Confirmed Pos. (HIV-2) No. Reactive/No. Tested | % | Confirmed Neg. Sera No. Reactive/No. Tested |
| GAG 2 | 9/19 | (47.4) | 2/2 | (100) | 0/4 |
| GAG 3 | 4/5 | (80) | N.D.[+] | | 0/2 |
| GAG 14 | 10/10 | (100) | N.D.[+] | | 0/2 |

[+] N.D. = not done

Example 10

Peptides P17-D and P17-F were synthesized as described in Example 1 and each peptide was coated at a concentration of 500 μg/ml onto microtiter plates and used in the ELISA described in Example 2, but substituting P17-D or P17-F for gp41A5 as the antigen. These peptides were also assayed against HIV-2 positive serum samples. The results are presented in Table 7.

HIV-2, a retrovirus related to, but not identical with, HIV-1, has been recently isolated from West African patients with AIDS who tested negative in ELISA tests using HIV-1 antigens.

It can be seen from the data in Tables 6 and 7 of Examples 9 and 10 that peptides GAG 2, P17-D and P17-F each contain epitopes recognized by antibodies to both HIV-1 and HIV-2. Thus these three peptides may be useful in diagnostics and vaccine compositions for both HIV-1 and HIV-2 infections.

TABLE 7

Immunologic Reactivity Of Antibodies In Serum Samples From HIV-1 and HIV-2 Patients With Peptides Corresponding To HIV-1 gag Gene Products

| Antigen Tested | Confirmed Pos. (HIV-1) No. Reactive/No. Tested | (%) | Confirmed Pos. (HIV-2) No. Reactive/No. Tested | (%) | Confirmed Neg. No. Reactive/No. |
|---|---|---|---|---|---|
| P17-D | 4/4 | (100) | 2/2 | (100) | 0/2 |
| P17-F | 5/5 | (100) | 1/1+ | | 0/2 |

+ weakly reactive

It is evident from the foregoing results that the novel synthetic peptides described herein which corresponding to the regions of immunologically important proteins of HIV-1, e.g., gp41 and gag gene products provide a superior, sensitive, and selective assay for the presence of antibodies to HIV-1.

## Claims

1. An antigenic peptide of the formula

X-Asp-Gln-Gln-Leu-Leu-Gly-Ile-Trp-Gly-Cys-Ser-Gly-
Lys-Leu-Ile-Cys-Thr-Thr-Ala-Val-Pro-Trp-Asn-Y-Z, wherein X is either a H of the amino terminal $NH_2$ group of the peptide or an additional amino acid bonded to the amino terminal $NH_2$ group of the peptide, the additional amino acid being selected to facilitate coupling of the peptide to a carrier protein; Y is absent or Cys; and Z is OH or $NH_2$.

2. An antigenic peptide of the formula

Asp-Gln-Gln-Leu-Leu-Gly-Ile-Trp-Gly-Cys-Ser-Gly-
Lys-Leu-Ile-Cys-Thr-Thr-Ala-Val-Pro-Trp-Asn-Cys-OH.

3. An antigenic peptide of the formula

X-Ala-Leu-Lys-Tyr-Trp-Trp-Asn-Leu-Leu-Gln-Tyr-
Trp-Ser-Gln-Glu-Leu-Lys-Asn-Ser-Ala-Val-Ser-Y-Z,

wherein X is either a H of the amino terminal $NH_2$ group of the peptide or an additional amino acid bonded to the amino terminal $NH_2$ group of the peptide, the additional amino acid being selected to facilitate coupling of the peptide to a carrier protein; Y, is absent or Cys; and Z is OH or $NH_2$.

4. An antigenic peptide of the formula

X-Lys-Asn-Ser-Ala-Val-Ser-Leu-Leu-Asn-Ala-Thr-
Ala-Ile-Ala-Val-Ala-Glu-Gly-Thr-Asp-Y-Z,

wherein X is either a H of the amino terminal $NH_2$ group of the peptide or an additional amino acid bonded to the amino terminal $NH_2$ group of the peptide, the additional amino acid being selected to facilitate coupling of the peptide to a carrier protein; Y, is absent or Cys; and Z is OH or $NH_2$.

5. An antigenic peptide of the formula

X-Thr-Ala-Val-Pro-Trp-Asn-Ala-Ser-Trp-Ser-Asn-Lys-
Ser-Leu-Glu-Gln-Ile-Trp-Asn-Asn-Met-Thr-Trp-Met-Y-Z,

wherein X is either a H of the amino terminal $NH_2$ group of the peptide or an additional amino acid bonded to the amino terminal $NH_2$ group of the peptide, the additional amino acid being selected to facilitate coupling of the peptide to a carrier protein; Y, is absent or Cys; and Z is OH or $NH_2$.

6. An antigenic peptide of the formula

X-Ile-Asn-Asn-Tyr-Thr-Ser-Leu-Ile-His-Ser-Leu-Ile-
Glu-Glu-Ser-Gln-Asn-Gln-Gln-Glu-Lys-Asn-Glu-Y-Z,

wherein X is either a H of the amino terminal $NH_2$ group of the peptide or an additional amino acid bonded to the amino terminal $NH_2$ group of the peptide, the additional amino acid being selected to facilitate coupling of the peptide to a carrier protein; Y, is absent of Cys; and Z is OH or $NH_2$.

7. An antigenic peptide of the formula

X-Pro-Arg-Thr-Leu-Asn-Ala-Trp-Val-Lys-Val-Val-Glu-
Glu-Lys-Ala-Phe-Ser-Pro-Glu-Val-Y-Z,

wherein X is either a H of the amino terminal $NH_2$ group of the peptide or an additional amino acid bonded to the amino terminal $NH_2$ group of the peptide, the additional amino acid being selected to facilitate coupling of the peptide to a carrier protein; Y, is absent or Cys; and Z is OH or $NH_2$.

8. An antigenic peptide of the formula

X-Val-Glu-Glu-Lys-Ala-Phe-Ser-Pro-Glu-Val-Ile-
Pro-Met-Phe-Ser-Ala-Leu-Ser-Glu-Gly-Y-Z,

wherein X is either a H of the amino terminal $NH_2$ group of the peptide or an additional amino acid bonded to the amino terminal $NH_2$ group of the peptide, the additional amino acid being selected to facilitate coupling of the peptide to a carrier protein; Y, is absent or Cys; and Z is OH or $NH_2$.

9. An antigenic peptide of the formula

X-Glu-Met-Met-Thr-Ala-Cys-Gln-Gly-Val-Gly-Gly-
Pro-Gly-His-Lys-Ala-Arg-Val-Leu-Ala-Glu-Y-Z,

wherein X is either a H of the amino terminal $NH_2$ group of the peptide or an additional amino acid bonded to the amino terminal $NH_2$ group of the peptide, the additional amino acid being selected to facilitate coupling of the peptide to a carrier protein; Y, is absent or Cys; and Z is OH or $NH_2$.

10. An antigenic peptide of the formula

X-Ser-Glu-Gly-Cys-Arg-Gln-Ile-Leu-Gly-Gln-Leu-Gln-
Pro-Ser-Leu-Gln-Thr-Gly-Ser-Glu-Glu-Leu-Y-Z,

wherein X is either a H of the amino terminal $NH_2$ group of the peptide or an additional amino acid bonded to the amino terminal $NH_2$ group of the peptide, the additional amino acid being selected to facilitate coupling of the peptide to a carrier protein; Y, is absent or Cys; and Z is OH or $NH_2$.

11. An antigenic peptide of the formula

X-Leu-Tyr-Cys-Val-His-Gln-Arg-Ile-Glu-Ile-Lys-
Asp-Thr-Lys-Glu-Ala-Leu-Asp-Lys-Ile-Y-Z,

wherein X is either a H of the amino terminal $NH_2$ group of the peptide or an additional amino acid bonded to the amino terminal $NH_2$ group of the peptide, the additional amino acid being selected to facilitate coupling of the peptide to a carrier protein; Y, is absent or Cys; and Z is OH or $NH_2$.

12. A method for detecting antibodies to HIV-1 in a sample comprising

contacting the sample with at least one peptide selected from the group consisting of

X-Asp-Gln-Gln-Leu-Leu-Gly-Ile-Trp-Gly-Cys-Ser-Gly-Lys-Leu-Ile-Cys-Thr-Thr-Ala-Val-Pro-Trp-Asn-Y-Z,

Asp-Gln-Gln-Leu-Leu-Gly-Ile-Trp-Gly-Cys-Ser-Gly-Lys-Leu-Ile-Cys-Thr-Thr-Ala-Val-Pro-Trp-Asn-Cys-OH,

X-Ala-Leu-Lys-Tyr-Trp-Trp-Asn-Leu-Leu-Gln-Tyr-Trp-Ser-Gln-Glu-Leu-Lys-Asn-Ser-Ala-Val-Ser-Y-Z,

X-Lys-Asn-Ser-Ala-Val-Ser-Leu-Leu-Asn-Ala-Thr-Ala-Ile-Ala-Val-Ala-Glu-Gly-Thr-Asp-Y-Z,

X-Thr-Ala-Val-Pro-Trp-Asn-Ala-Ser-Trp-Ser-Asn-Lys-Ser-Leu-Glu-Gln-Ile-Trp-Asn-Asn-Met-Thr-Trp-Met-Y-Z,

X-Ile-Asn-Asn-Tyr-Thr-Ser-Leu-Ile-His-Ser-Leu-Ile-Glu-Glu-Ser-Gln-Asn-Gln-Gln-Glu-Lys-Asn-Glu-Y-Z,

X-Pro-Arg-Thr-Leu-Asn-Ala-Trp-Val-Lys-Val-Val-Glu-Glu-Lys-Ala-Phe-Ser-Pro-Glu-Val-Y-Z,

X-Val-Glu-Glu-Lys-Ala-Phe-Ser-Pro-Glu-Val-Ile-Pro-Met-Phe-Ser-Ala-Leu-Ser-Glu-Gly-Y-Z,

X-Glu-Met-Met-Thr-Ala-Cys-Gln-Gly-Val-Gly-Gly-Pro-Gly-His-Lys-Ala-Arg-Val-Leu-Ala-Glu-Y-Z,

X-Ser-Glu-Gly-Cys-Arg-Gln-Ile-Leu-Gly-Gln-Leu-Gln-Pro-Ser-Leu-Gln-Thr-Gly-Ser-Glu-Glu-Leu-Y-Z,

and

X-Leu-Tyr-Cys-Val-His-Gln-Arg-Ile-Glu-Ile-Lys-Asp-Thr-Lys-Glu-Ala-Leu-Asp-Lys-Ile-Y-Z,

wherein X is either a H of the amino terminal $NH_2$ group of the peptide or an additional amino acid bonded to the amino terminal $NH_2$ group of the peptide, the additional amino acid being selected to facilitate coupling of the peptide to a carrier protein; Y, is absent or Cys; and Z is OH or $NH_2$, under conditions such that an immunological complex will form between the peptide and antibodies to HIV-1 if such antibodies are present in the sample and

measuring the formation if any of the immunological complex to determine the presence of antibodies to HIV-1 in the sample.

13. A method for detecting of antibodies to HIV-1 in a sample comprising

contacting the sample with a peptide having the amino acid sequence Asp-Gln-Gln-Leu-Leu-Gly-Ile-Trp-Gly-Cys-Ser-Gly-Lys-Leu-Ile-Cys-Thr-Thr-Ala-Val-Pro-Trp-Asn-Cys-OH.

under conditions such that an immunological complex will form between the peptide and antibodies to HIV-1 if such antibodies are present in the sample and

measuring the formation if any of the immunological complex to determine the presence of antibodies to HIV-1 in the sample.

14. A composition for eliciting the production of antibodies against HIV-1 in animals including man comprising an immunogenically effective amount of at least one peptide selected from the group consisting of

X-Asp-Gln-Gln-Leu-Leu-Gly-Ile-Trp-Gly-Cys-Ser-Gly-Lys-Leu-Ile-Cys-Thr-Thr-Ala-Val-Pro-Trp-Asn-Y-Z,

Asp-Gln-Gln-Leu-Leu-Gly-Ile-Trp-Gly-Cys-Ser-Gly-Lys-Leu-Ile-Cys-Thr-Thr-Ala-Val-Pro-Trp-Asn-Cys-OH,

X-Ala-Leu-Lys-Tyr-Trp-Trp-Asn-Leu-Leu-Gln-Tyr-Trp-Ser-Gln-Glu-Leu-Lys-Asn-Ser-Ala-Val-Ser-Y-Z,

X-Lys-Asn-Ser-Ala-Val-Ser-Leu-Leu-Asn-Ala-Thr-Ala-Ile-Ala-Val-Ala-Glu-Gly-Thr-Asp-Y-Z,

X-Thr-Ala-Val-Pro-Trp-Asn-Ala-Ser-Trp-Ser-Asn-Lys-Ser-Leu-Glu-Gln-Ile-Trp-Asn-Asn-Met-Thr-Trp-Met-Y-Z,

X-Ile-Asn-Asn-Tyr-Thr-Ser-Leu-Ile-His-Ser-Leu-Ile-Glu-Glu-Ser-Gln-Asn-Gln-Gln-Glu-Lys-Asn-Glu-Y-Z,

X-Pro-Arg-Thr-Leu-Asn-Ala-Trp-Val-Lys-Val-Val-Glu-Glu-Lys-Ala-Phe-Ser-Pro-Glu-Val-Y-Z,

X-Val-Glu-Glu-Lys-Ala-Phe-Ser-Pro-Glu-Val-Ile-Pro-Met-Phe-Ser-Ala-Leu-Ser-Glu-Gly-Y-Z,

X-Glu-Met-Met-Thr-Ala-Cys-Gln-Gly-Val-Gly-Gly-Pro-Gly-His-Lys-Ala-Arg-Val-Leu-Ala-Glu-Y-Z,

X-Ser-Glu-Gly-Cys-Arg-Gln-Ile-Leu-Gly-Gln-Leu-Gln-Pro-Ser-Leu-Gln-Thr-Gly-Ser-Glu-Glu-Leu-Y-Z,

and

X-Leu-Tyr-Cys-Val-His-Gln-Arg-Ile-Glu-Ile-Lys-Asp-Thr-Lys-Glu-Ala-Leu-Asp-Lys-Ile-Y-Z,

wherein X is either a H of the amino terminal $NH_2$ group of the peptide or an additional amino acid bonded to the amino terminal $NH_2$ group of the peptide, the additional amino acid being selected to facilitate coupling of the peptide to a carrier protein; Y, is absent or Cys; and Z is OH or $NH_2$, and a physiologically acceptable carrier.

15. A composition for eliciting the production of antibodies against HIV-1 in animals including man comprising an immunogenically effective amount of a peptide of formula

Asp-Gln-Gln-Leu-Leu-Gly-Ile-Trp-Gly-Cys-Ser-Gly-Lys-Leu-Ile-Cys-Thr-Thr-Ala-Val-Pro-Trp-Asn-Cys-OH,

and a physiologically acceptable carrier.

16. A method for eliciting antibodies to HIV-1 in an animal including man comprising administering to the animal an immunogenically effective amount of at least one peptide selected from the group consisting of

X-Asp-Gln-Gln-Leu-Leu-Gly-Ile-Trp-Gly-Cys-Ser-Gly-Lys-Leu-Ile-Cys-Thr-Thr-Ala-Val-Pro-Trp-Asn-Y-Z,

Asp-Gln-Gln-Leu-Leu-Gly-Ile-Trp-Gly-Cys-Ser-Gly-Lys-Leu-Ile-Cys-Thr-Thr-Ala-Val-Pro-Trp-Asn-Cys-OH,

X-Ala-Leu-Lys-Tyr-Trp-Trp-Asn-Leu-Leu-Gln-Tyr-
Trp-Ser-Gln-Glu-Leu-Lys-Asn-Ser-Ala-Val-Ser-Y-Z,

X-Lys-Asn-Ser-Ala-Val-Ser-Leu-Leu-Asn-Ala-Thr-
Ala-Ile-Ala-Val-Ala-Glu-Gly-Thr-Asp-Y-Z,

X-Thr-Ala-Val-Pro-Trp-Asn-Ala-Ser-Trp-Ser-Asn-Lys-
Ser-Leu-Glu-Gln-Ile-Trp-Asn-Asn-Met-Thr-Trp-Met-Y-Z,

X-Ile-Asn-Asn-Tyr-Thr-Ser-Leu-Ile-His-Ser-Leu-Ile-
Glu-Glu-Ser-Gln-Asn-Gln-Gln-Glu-Lys-Asn-Glu-Y-Z,

X-Pro-Arg-Thr-Leu-Asn-Ala-Trp-Val-Lys-Val-Val-Glu-
Glu-Lys-Ala-Phe-Ser-Pro-Glu-Val-Y-Z,

X-Val-Glu-Glu-Lys-Ala-Phe-Ser-Pro-Glu-Val-Ile-
Pro-Met-Phe-Ser-Ala-Leu-Ser-Glu-Gly-Y-Z,

X-Glu-Met-Met-Thr-Ala-Cys-Gln-Gly-Val-Gly-Gly-
Pro-Gly-His-Lys-Ala-Arg-Val-Leu-Ala-Glu-Y-Z,

X-Ser-Glu-Gly-Cys-Arg-Gln-Ile-Leu-Gly-Gln-Leu-Gln-
Pro-Ser-Leu-Gln-Thr-Gly-Ser-Glu-Glu-Leu-Y-Z,

and

X-Leu-Tyr-Cys-Val-His-Gln-Arg-Ile-Glu-Ile-Lys-
Asp-Thr-Lys-Glu-Ala-Leu-Asp-Lys-Ile-Y-Z,

wherein X is either a H of the amino terminal $NH_2$ group of the peptide or an additional amino acid bonded to the amino terminal $NH_2$ group of the peptide, the additional amino acid being selected to facilitate coupling of the peptide to a carrier protein; Y, is absent or Cys; and Z is OH or $NH_2$, and a physiologically acceptable carrier.

17. A method for eliciting antibodies to HIV-1 in an animal including man comprising administering to the animal an immunogenically effective amount of a peptide of formula

Asp-Gln-Gln-Leu-Leu-Gly-Ile-Trp-Gly-Cys-Ser-Gly-
Lys-Leu-Ile-Cys-Thr-Thr-Ala-Val-Pro-Trp-Asn-Cys-OH,

and a physiologically acceptable carrier.

FIG. 1

0284587

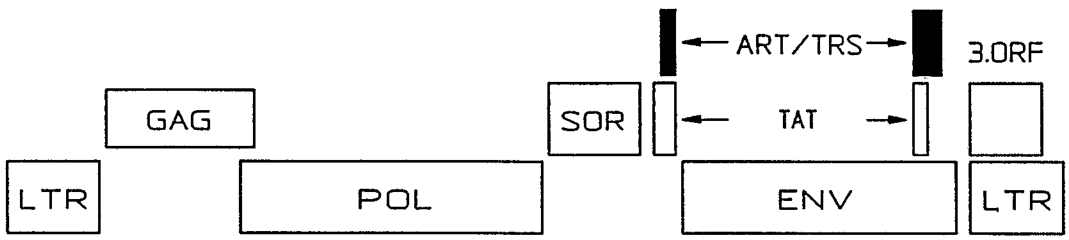

FIG. 2

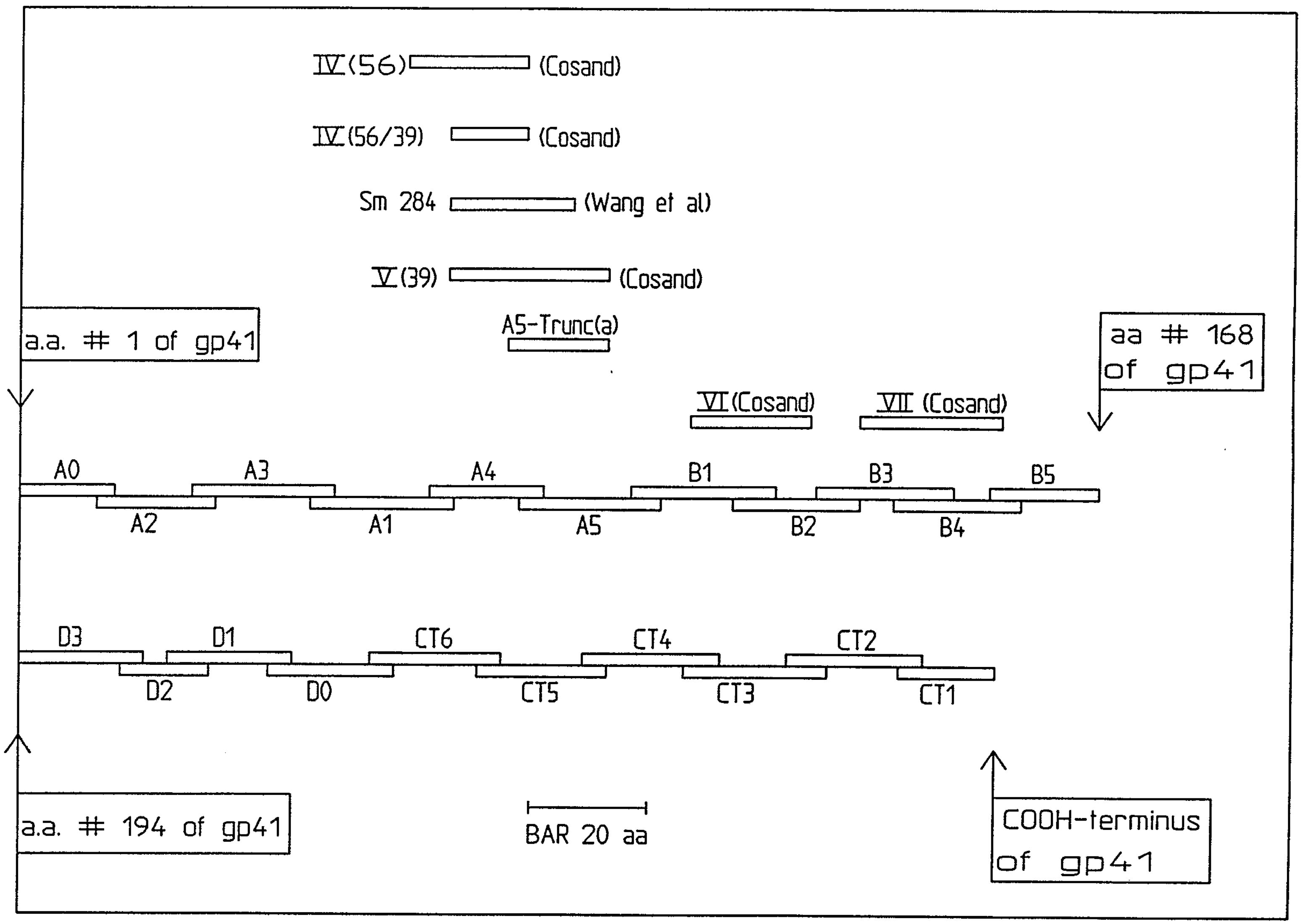

IV (56)
(Cosand)
IV (56/39)
(Cosand)
Sm 284
(Wang et al)
V (39)
(Cosand)
A5-Trunc(a)
VI (Cosand)
VII (Cosand)
a.a. # 1 of gp41
aa # 168 of gp41
A0
A3
A4
B1
B3
B5
A2
A1
A5
B2
B4
D3
D1
CT6
CT4
CT2
D2
D0
CT5
CT3
CT1
a.a. # 194 of gp41
BAR 20 aa
COOH-terminus of gp41

# FIG 3

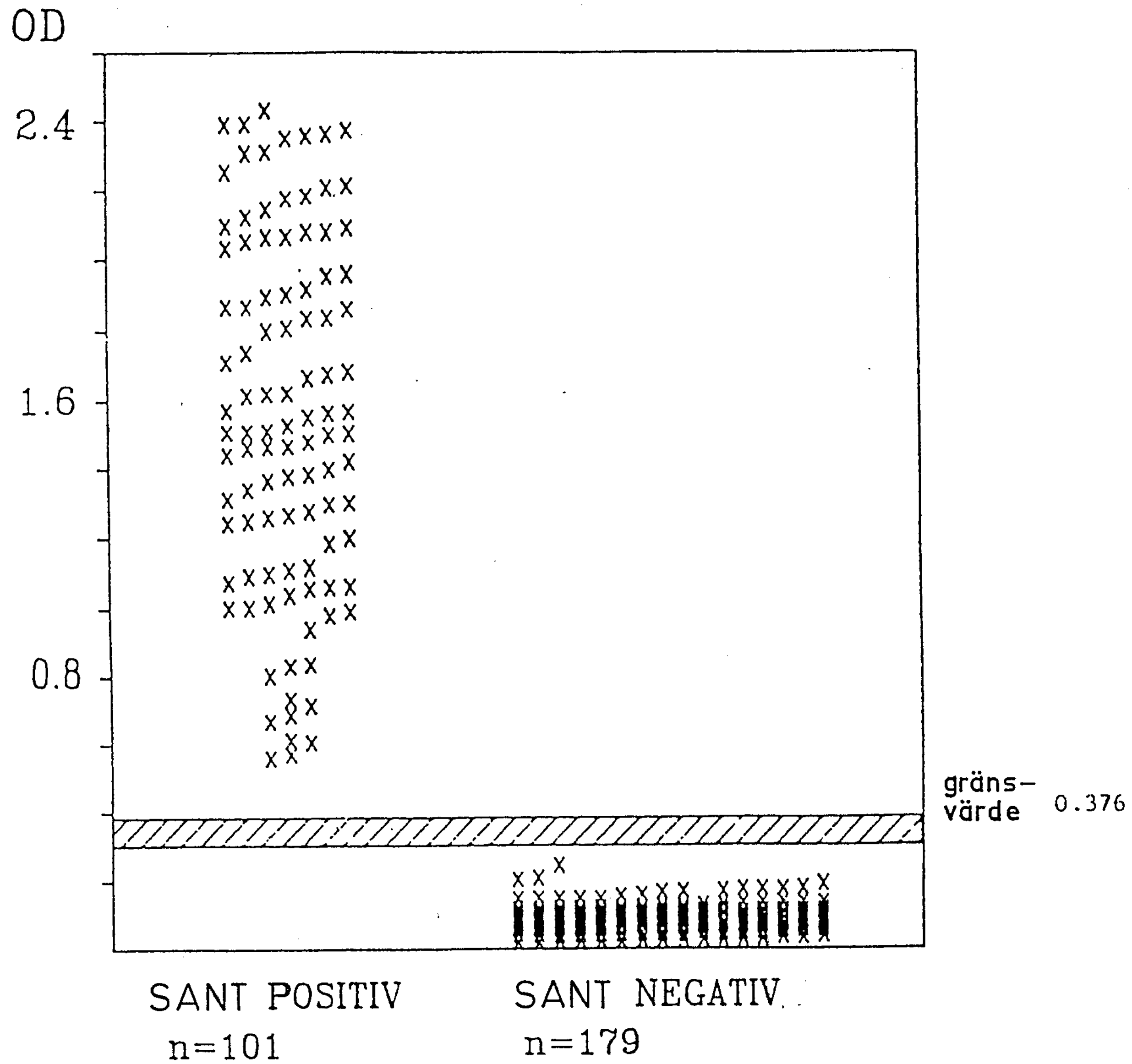
OD
2.4
1.6
0.8
gräns-
värde
0.376
SANT POSITIV
n=101
SANT NEGATIV
n=179